# EUROPEAN PATENT SPECIFICATION

(11) **EP 4 111 340 B1**
(45) Date of publication and mention of the grant of the patent: **29.07.2026**
(21) Application number: 21760874.4
(22) Date of filing: 26.02.2021
(51) Int. Cl.: G06F 21/56, A61M 5/14, A61M 5/142, G21G 4/08

(54) **RADIOPHARMACEUTICAL INFUSION SYSTEM**
RADIOPHARMAZEUTISCHES INFUSIONSSYSTEM
SYSTÈME DE PERFUSION RADIOPHARMACEUTIQUE

(30) Priority: 28.02.2020 US 202062983498 P
(43) Date of publication of application: 04.01.2023
(73) Proprietor: JUBILANT DRAXIMAGE INC., Montréal, Québec H9H414 (CA)
(72) Inventor: NANDI, Indranil, Yardley, PA 19067 (US)
(74) Representative: Pons IP
(86) International application number: PCT/US2021/020086
(87) International publication number: WO 2021/174122

(56) References cited:
- US-A- 4 160 910
- US-A1- 2002 166 067
- US-A1- 2005 108 557
- US-A1- 2006 004 737
- US-A1- 2010 312 039
- US-A1- 2011 023 115
- US-A1- 2011 023 115
- US-A1- 2011 247 071
- US-A1- 2015 228 368
- US-A1- 2017 363 750
- US-A1- 2018 296 751
- US-A1- 2019 307 949

## Description

### TECHNICAL FIELD

The present disclosure relates to a field of nuclear medicine and, in particular, to radiopharmaceutical infusion systems for the generation and administration of a radiopharmaceutical composition for diagnosis and/or treatment of a disease.

### BACKGROUND

The radiopharmaceuticals play a pivotal role in the diagnosis and patient management of various diseased conditions. The radioisotopes for pharmaceutical use are produced either by nuclear bombardment in cyclotron or in-situ by employing radioisotope generators at the site of use.

Radiopharmaceutical infusion systems generate and administer radioisotopes to patients for diagnosis or treatment of diseases. Radiopharmaceutical infusion systems are commonly used in radiopharmacies, hospitals and research institutes. Use of such systems is increasing substantially for treatment or diagnostic purposes because of advancements in radiopharmaceutical generation, development and administration technology.

Radiopharmaceutical infusion systems include various components like controller, pump, valves, sensors, a radiopharmaceutical source, cabinet, shielded assembly, tubing circuit, activity detector, dose calibrator, computer, internal memory, user interface and other accessories. Controller is the key component of the radiopharmaceutical infusion system which controls most of the device operations. Radiopharmaceutical infusion systems may be connected to one or more equipment or networks like imaging systems, hospital networks, pharmacies, servers, remote computers, iPads, mobile tablets, mobile phones, watches or other electronic devices. The radiopharmaceutical infusion system may be connected to such equipment *via* wired or wireless connections like Local Area Network (LAN), Communication ports, Wireless Fidelity (WIFI), Universal Serial Bus (USB), Bluetooth, Cables, Compact Disc, Digital Video Disc or combinations thereof. US 2011/023115A1 discloses a radiopharmaceutical infusion system according to the state of the art.

The radiopharmaceutical infusion systems may carry a high risk of radiation hazard and exposure to patients or operator. Illegal access to controlling components like computer, software or internal memory of infusion system could lead to a hazardous undesirable situation.

In recent years, regulatory agencies have raised several concerns over cybersecurity of the medical devices. United States Food and Drug Administration (USFDA) has emphasized the importance of effective cybersecurity to ensure medical device functionality and safety with the increasing use of wireless, internet and network connected devices, portable media and the frequent electronic exchange of medical device related health information. Further, cybersecurity threats to the healthcare sector have become frequent and severe. Cybersecurity related incidents have rendered medical devices and hospital networks inoperable disrupting the delivery of patient care across healthcare facilities in the US and globally. Such cyberattacks can delay diagnosis and/or treatment.

Due care is taken by the healthcare professionals to isolate radiopharmaceutical infusion system from network, internet or other connections however, sometimes it becomes necessary to connect such systems to other devices or a network *via* various channels for exchange of information with healthcare professionals and for maintenance or updating of the system. The increased use of wireless, internet coupled to an infusion system has made such systems vulnerable to cybersecurity threats.

Radiopharmaceutical elution/infusion systems may carry radiation hazards for the patients and operators hence cybersecurity is the need of the hour in view of increasing connectivity obligations and imminent cybersecurity threats. Thus, there is a need for ensuring cybersecurity of radiopharmaceutical infusion systems.

### SUMMARY

The present disclosure provides cybersecurity mechanisms for radiopharmaceutical infusion systems. The invention is defined according to independent claims 1 and 13 and dependent claims 2-12 defining further embodiments.

It is an object of the present disclosure to enhance safety of the radiopharmaceutical infusion systems used for generation and administration of radiopharmaceutical into patients for diagnosis and/or treatment.

It is an object of the present disclosure to enhance the safety of rubidium-82 infusion system.

It is an object of the present disclosure, wherein the radiopharmaceutical infusion system controller is configured to detect an unauthorized connection and/or malware in the system, network or connected devices.

It is an object of the present disclosure to enhance the safety of radiopharmaceutical infusion system, wherein the controller is configured to ensure that the radiopharmaceutical infusion system is free of an unauthorized connection and/or malware.

It is an object of the present disclosure to enhance safety of radiopharmaceutical infusion systems by scanning systems, network or connected devices for an unauthorized connection and/or malware during daily quality control of infusion system or prior to system initiation.

It is an object of the present disclosure to enhance the cybersecurity of radiopharmaceutical infusion systems, wherein the controller is configured to scan the system, network or connected devices during daily quality control for unauthorized connection and/or malware prior to patient elution to ensure the system is free from an unauthorized connection and/or malware.

It is an object of the present disclosure to enhance cybersecurity of the radiopharmaceutical infusion system, wherein the controller is configured to scan the system, network or connected devices for an unauthorized connection and/or malware continuously during the operation of infusion system to ensure the system is free from unauthorized connection and/or malware.

It is an object of the present disclosure to enhance cybersecurity of the radiopharmaceutical infusion system, wherein the controller is configured to scan the system, network or connected devices for an unauthorized connection and/or malware at predetermined intervals of time to ensure system is free from unauthorized connection and/or malware.

It is an object of the present disclosure to enhance the cybersecurity of the radiopharmaceutical infusion system, wherein the controller is configured to scan the computer, network or connected devices for an unauthorized connection and/or malware prior to patient elution and ensure the system is free from unauthorized connection and/or malware.

It is an object of the present disclosure to enhance safety of radiopharmaceutical infusion systems by scanning the system, network or connected devices for an unauthorized connection and/or malware in the background or overnight and alert the operator about any malware or unauthorized connection before starting the system.

It is an object of the present disclosure, wherein the radiopharmaceutical infusion system controller is configured to alert the operator of an unauthorized connection and/or malware.

It is an object of the present disclosure, wherein the radiopharmaceutical infusion system controller is configured to force the system into safe mode in case of an unauthorized connection and/or malware is detected.

It is an object of the present disclosure, wherein the radiopharmaceutical infusion system controller is configured to perform automatic data backup to a remote secure device, network or data cloud to safeguard the data if any cyber threat is detected.

It is an object of the present disclosure, wherein the radiopharmaceutical infusion system controller is configured to stop the operation of the system in case any threat of unauthorized connection and/or malware is detected and prevents operations till the threat is neutralized.

The present disclosure concerns any of the following items:

A radiopharmaceutical infusion system comprising a source of the radiopharmaceutical, a pump, an eluant in a reservoir, a waste bottle, a tubing line, a sensor, a valve and a controller, wherein the source of radiopharmaceutical comprises a generator column containing a parent radioisotope that decays into a daughter radioisotope, wherein the pump is able to infuse an eluant through the generator column and provide a sample, wherein the controller is configured to perform quality control tests on the system comprising:
a) a calibration of the sample to determine the daughter isotope activity;
b) a breakthrough test that measures the parent radioisotope activity in the sample; and
c) a connection scan on the system, on a network connected to the system or on a device connected to the system in order to confirm absence of an unauthorized connection.

A radiopharmaceutical infusion system comprising a source of a radiopharmaceutical, a pump, an eluant in a reservoir, a waste bottle, a tubing line, a sensor, a valve and a controller, wherein the source of radiopharmaceutical comprises a generator column containing a parent radioisotope that decays into a daughter radioisotope, wherein the pump is able to infuse an eluant through the generator column and provide a sample, wherein the controller is configured to perform quality control tests on the system comprising:
a) a calibration of the sample to determine the daughter isotope activity;
b) a breakthrough test that measures the parent radioisotope activity in the sample; and
c) a malware scan on the system, on a network connected to the system or on a device connected to the system in order to confirm absence of a malware.

A radiopharmaceutical infusion system comprising a source of radiopharmaceutical, a pump, an eluant in a reservoir, a waste bottle, a tubing line, a sensor, a valve and a controller, wherein the source of radiopharmaceutical comprises a generator column containing a parent radioisotope that decays into a daughter radioisotope, wherein the pump is able to infuse an eluant through the generator column and provide a sample, wherein the controller is configured to perform quality control tests on the system comprising:
a) a calibration of the sample to determine the daughter isotope activity;
b) a breakthrough test that measures the parent radioisotope activity in the sample;
c) a connection scan on the system, on a network connected to the system or on a device connected to the system in order to confirm absence of an unauthorized connection; and
d) a malware scan on the system, on a network connected to the system or on a device connected to the system in order to confirm absence of a malware.

A radiopharmaceutical infusion system comprising a source of radiopharmaceutical, a pump, an eluant in a reservoir, a waste bottle, a tubing line, a sensor, a valve and a controller, wherein the source of radiopharmaceutical comprises a generator column containing a parent radioisotope that decays into a daughter radioisotope, wherein the pump is able to infuse an eluant through the generator column and provide a sample, wherein the controller is configured to perform quality control tests on the system comprising:
a) a connection scan on the system, on a network connected to the system or on a device connected to the system in order to confirm absence of an unauthorized connection;
b) a malware scan on the system, on a network connected to the system or on a device connected to the system in order to confirm absence of a malware;
c) a calibration of the sample to determine the daughter isotope activity; and
d) a breakthrough test that measures the parent radioisotope activity in the sample.

The radiopharmaceutical infusion system, wherein the controller is configured to allow operation of the system after the breakthrough test is found to be acceptable.

The radiopharmaceutical infusion system, wherein the controller is configured to allow operation of the system after the quality control is found to be acceptable.

The radiopharmaceutical infusion system, wherein the system is automated.

The radiopharmaceutical infusion system, further comprising additional quality control test comprising verification of the status of the generator column, a flow rate of the eluant, a pump speed, a leakage of the eluant, the type of the eluant, a volume in the waste bottle, the volume of eluant in the reservoir, the volume of eluant that has passed through the generator column the pressure in the tubing line, the activity of the parent isotope and the daughter isotope, sensor functioning and valve functioning.

The radiopharmaceutical infusion system, wherein the quality control tests are performed automatically.

The radiopharmaceutical infusion system, wherein the additional quality control tests are performed automatically.

The radiopharmaceutical infusion system, wherein the source of radiopharmaceutical comprises a radiopharmaceutical generator loaded with parent isotope which elutes a daughter isotope.

The radiopharmaceutical infusion system wherein radiopharmaceutical is selected from ¹⁸F, ⁸²Rb, ¹⁵O, ¹³N, ⁶⁸Ga, ⁹⁹Mo/^{99m}Tc, ⁹⁰Sr/⁹⁰Y, ⁸²Sr/⁸²Rb, ¹⁸⁸W/¹⁸⁸Re, ⁶⁸Ge/⁶⁸Ga, ⁴²Ar/⁴²K, ⁴⁴Ti/⁴⁴Sc, ⁵²Fe/^{52m}Mn, ⁷²Se/⁷²As, ⁸³Rb/^{83m}Kr, ¹⁰³Pd/^{103m}Rh, ¹⁰⁹Cd/^{109m}Ag, ¹¹³Sn/^{113m}In, ¹¹⁸Te/¹¹⁸Sb, ¹³²Te/¹³²I, ¹³⁷Cs/^{137m}Ba, ¹⁴⁰Ba/¹⁴⁰La, ¹³⁴Ce/¹³⁴La, ¹⁴⁴Ce/¹⁴⁴Pr, ¹⁴⁰Nd/¹⁴⁰Pr, ¹⁶⁶Dy/¹⁶⁶Ho, ¹⁶⁷Tm/^{167m}Er, ¹⁷²Hf/¹⁷²Lu, ¹⁷⁸W/¹⁷⁸Ta, ¹⁹¹Os/ ^{191m}Ir, ¹⁹⁴Os/¹⁹⁴Ir, ²²⁶Ra/²²²Rn and ²²⁵Ac/²¹³Bi.

The radiopharmaceutical infusion system, wherein daughter isotope is selected from ¹⁸F, ⁸²Rb, ¹⁵O, ¹³N and ⁶⁸Ga.

The radiopharmaceutical infusion system, wherein the source of radiopharmaceutical comprises a rubidium-82 generator.

The radiopharmaceutical infusion system, wherein malware comprises a virus, a worm, a trojan horse, a ransomware, a spyware, an adware, a scareware, a keylogger, a mirai, reaper, a emotet, a bot, a botnet, a rootkit or combinations thereof.

The radiopharmaceutical infusion system, comprises intrusion detection system, a firewall or a sandboxing wherein said intrusion detection system, firewall or sandboxing is embedded in the system, network or located remotely.

The radiopharmaceutical infusion system, wherein the intrusion detection system comprises a firewall, an anti-virus, artificial intelligence based software, a learning machine or a deep learning based software or hardware.

The radiopharmaceutical infusion system, wherein the controller is further configured to perform a malware scan and/or an unauthorized connection scan continuously.

The radiopharmaceutical infusion system, wherein the controller is further configured to perform a malware and/or an unauthorized connection scan at a predetermined interval of time.

The radiopharmaceutical infusion system, wherein the controller is further configured to perform a malware scan and/or an unauthorized connection scan after an elution and generator column is recharging.

The radiopharmaceutical infusion system, wherein upon detection of an attempt to connect the system, the controller is configured to; a) provide an alert to a user about said such attempt that requests confirmation of the authenticity of the said attempt, and b) stops the system from operating in case the user fails to authenticate the detected attempt to connect the system.

The radiopharmaceutical infusion system, wherein the controller is configured to stop the system from operating in case a malware is detected by the malware scan until the malware is neutralized.

The radiopharmaceutical infusion system, wherein the controller is configured to repeat the quality control tests in case a malware or an unauthorized connection is detected on the system.

A radiopharmaceutical infusion system comprising a source of the radiopharmaceutical, a pump, an eluant in a reservoir, a waste bottle, a tubing line, a sensor, a valve and a controller, wherein the source of radiopharmaceutical comprises a generator column containing a parent radioisotope that decays into a daughter radioisotope, wherein the pump is able to infuse an eluant through the generator column and provide a sample, wherein the system is configured to perform malware scan and/or unauthorized connection scan in the background during operation of the system.

A radiopharmaceutical infusion system, wherein the controller is configured to allow the patient infusion in case the system is found to be free of malware or unauthorized connection.

A radiopharmaceutical infusion system comprising a source of radiopharmaceutical, a pump, an eluant in a reservoir, a waste bottle, a tubing line, a sensor, a valve and a controller, wherein the source of radiopharmaceutical comprises a generator column containing a parent radioisotope that decays into a daughter radioisotope, wherein the pump is able to infuse an eluant through the generator column and provide a sample, wherein the controller is configured to perform:
a) a connection scan on the system, on a network connected to the system, or on a device connected to the system in order to confirm absence of an unauthorized connection; and
b) a malware scan on the system, on a network connected to the system or on a device connected to the system in order to confirm absence of a malware;
wherein the controller is configured to allow system operation once the absence of malware and/or an unauthorized connection is confirmed.

The radiopharmaceutical infusion system, wherein the malware and connection scan is performed overnight and results are displayed prior to breakthrough and calibration testing performed before starting patient infusion. The results are displayed prior to start of the mandatory quality control.

### BRIEF SUMMARY OF THE DRAWINGS

Further features and advantages of the present disclosure will become apparent from the following detailed description, taken in combination with the appended drawings, in which:
**Fig.1** is a block diagram schematically illustrating principal elements of a rubidium elution system in accordance with an embodiment according to the present disclosure.
**Fig.2** is a block diagram schematically illustrating principal elements of a rubidium elution system in accordance with another embodiment according to the present disclosure.

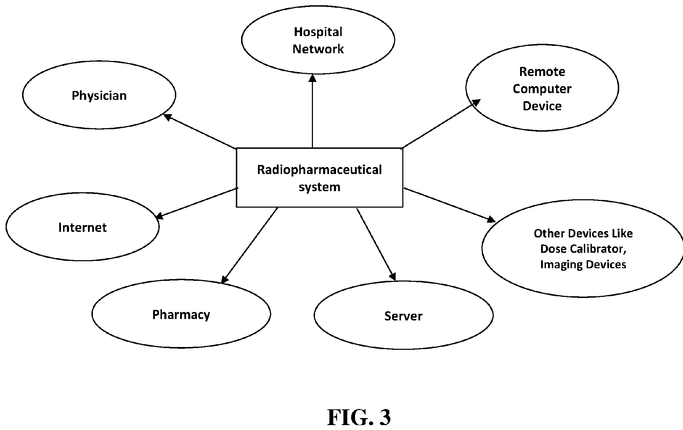

**Fig.3** illustrates the connections of the device in accordance with another embodiment according to the present disclosure.

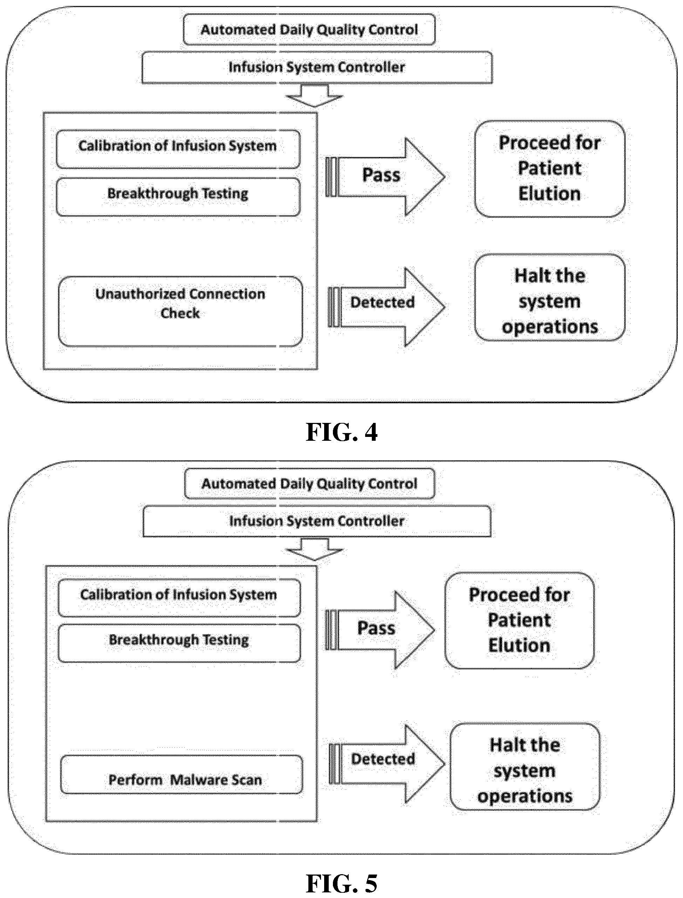

**Fig.4** illustrates steps of automated quality control of rubidium elution system in accordance with another embodiment according to the present disclosure.
**Fig.5** illustrates steps of automated quality control of rubidium elution system in accordance with another embodiment according to the present disclosure.

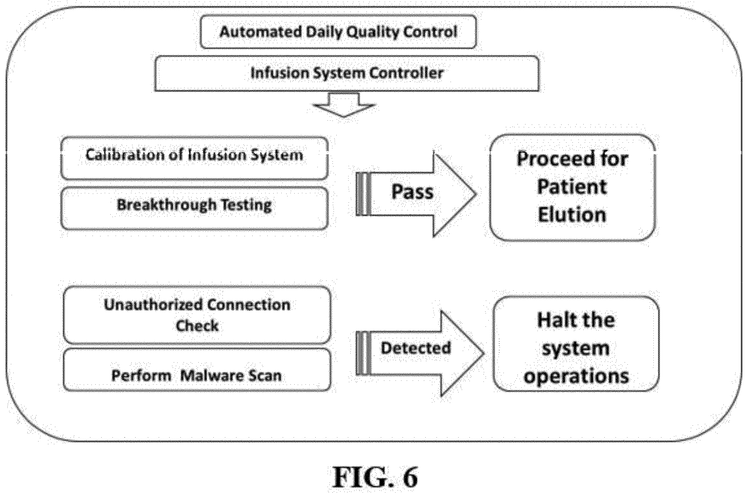

**Fig.6** illustrates steps of automated quality control of rubidium elution system in accordance with another embodiment according to the present disclosure.

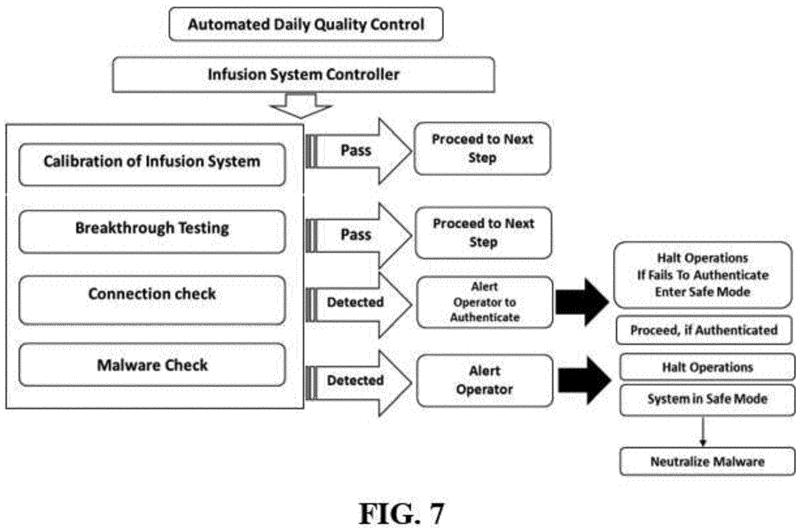

**Fig.7** illustrates steps of automated quality control of rubidium elution system including alerts and safety procedure in accordance with another embodiment according to the present disclosure.

### DETAILED DESCRIPTION

The present inventive subject matter can be more readily understood by reading the following detailed description of representative embodiments.

As used herein, "radiopharmaceutical infusion system" according to the present invention comprises equipment for the generation and/or administration of radiopharmaceuticals in patients for diagnosis or treatment of a disease.

As used herein, "network" is a group of two or more computer systems linked together. There are many types of computer networks like local area networks (LANs), Wide area networks (WANs), Campus area networks (CANs), Metropolitan-area networks (MANs), Home area networks (HANs). The network may include any hardware or software for connecting the computing device in a communicating relationship with other resources through the network. This may include remote resources accessible through the internet as well as local resources using physical connections like ethernet, radio frequency communications like WiFi, optical communications like fiber optics, infrared, ultrasonic communications or any combination of these or communications through any other media that might be used to carry data between the infusion system and other devices. The network interface may include a modem, router, a network card, an infrared transceiver, a radio frequency transceiver, a near field communications interface, a radio-frequency identification tag reader or the like. The device may be connected to the internet of things for various purposes like tracking of device location using sensors attached to the device for making it visible to hospital staff. In an embodiment, the infusion system may be equipped with various sensors which include but are not limited to tracking of consumables like diluent, eluant, leakage sensor, volume overflow sensors, pressure sensors to detect pressure in the tubing or system, touch sensors, temperature sensor, humidity sensors, patient movement sensors during elution or imaging procedure, emergency or alert messages to the operator, device maintenance requirements, remaining life of radiopharmaceutical generator, information about automated quality control procedures, completion of the radiopharmaceutical infusion, connection attempt to the device. The connected devices may include computers, personal computers, laptops, iPads, mobile tablets, mobile phones, wearable devices, watches and the like.

As used herein, the term "radiopharmaceutical" comprises radioisotopes used in the field of pharmaceuticals for treatment, diagnosis or imaging. The radioisotopes according to the present invention comprises ¹⁸F, ⁸²Rb, ¹⁵O, ¹³N, ⁶⁸Ga, ⁹⁹Mo/^{99m}Tc, ⁹⁰Sr/⁹⁰Y, ⁸²Sr/⁸²Rb, ¹⁸⁸W/¹⁸⁸Re, ⁶⁸Ge/⁶⁸Ga, ⁴²Ar/⁴²K, ⁴⁴Ti/⁴⁴Sc, ⁵²Fe/^{52m}Mn, ⁷²Se/⁷²As, ⁸³Rb/^{83m}Kr; ¹⁰³Pd/^{103m}Rh, ¹⁰⁹Cd/^{109m}Ag, ¹¹³Sn/^{113m}In, ¹¹⁸Te/¹¹⁸Sb, ¹³²Te/¹³²I, ¹³⁷Cs/^{137m}Ba, ¹⁴⁰Ba/¹⁴⁰La, ¹³⁴Ce/¹³⁴La, ¹⁴⁴Ce/¹⁴⁴Pr, ¹⁴⁰Nd/¹⁴⁰Pr, ¹⁶⁶Dy/¹⁶⁶Ho, ¹⁶⁷Tm/^{167m}Er, ¹⁷²Hf/¹⁷²Lu, ¹⁷⁸W/¹⁷⁸Ta, ¹⁹¹Os/ ^{191m}Ir, ¹⁹⁴Os/¹⁹⁴Ir, ²²⁶Ra/²²²Rn and ²²⁵Ac/²¹³Bi.

As used herein, the "radiopharmaceutical infusion system" comprises a system for generation and infusion of the radiopharmaceutical in a patient for Positron Emission Tomography (PET) or Single Photon Emission Computed Tomography (SPECT) imaging. The radiopharmaceutical can be generated by elution from the generator. Positron emission tomography is a nuclear medicine functional imaging technique that is used to observe metabolic processes in the body as an aid for diagnosis and/or treatment of disease. The system detects pairs of gamma rays emitted indirectly by a positron-emitting radioligand. Preferred PET agents include but are not limited to Fluorine-18, Rubidium-82, Oxygen-15, Carbon-11 and Copper-64. Three dimensional images of tracer concentration within the body are then constructed by computer analysis.

In another aspect of the present invention, the radiopharmaceutical infusion system comprises one or more components selected from a controller connected to on board or off board computer, pump, valves, sensors, generator assembly, cabinet, shielded assembly, radiopharmaceutical generator, tubing circuit, activity detector, dose calibrator, internal memory, screen, eluant, reservoir, cabinet and other accessories. The infusion system according to the present invention comprises a rubidium infusion system. ⁸²Rb is a daughter isotope generated by radioactive decay of parent isotope ⁸²Sr bound to ion exchange material in a shielded radioisotope generator called the source of radiopharmaceutical in the radiopharmaceutical infusion system.

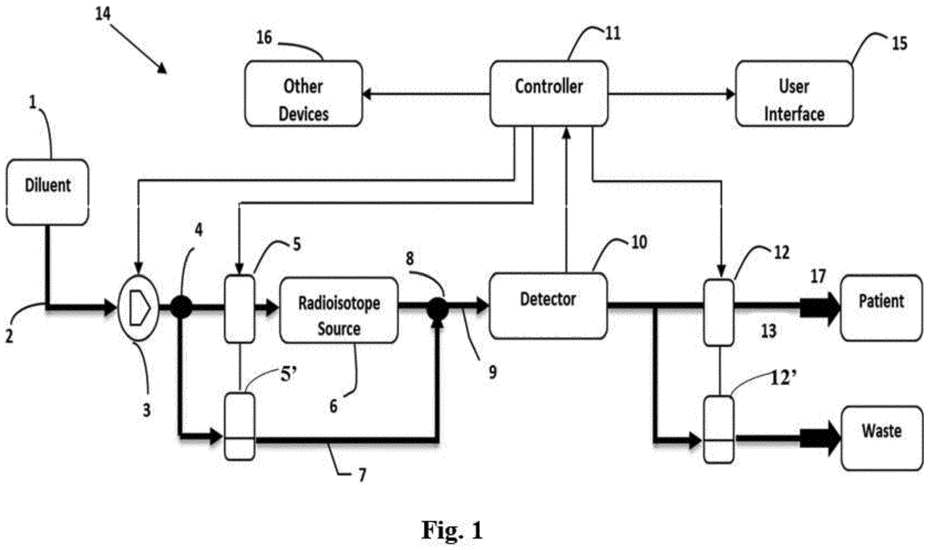

The rubidium infusion system comprises the components as described in **FIG. 1****.** In an embodiment, the elution system comprises reservoir 1 of sterile eluant or diluent solution; a pump 3 used for drawing eluant from the reservoir 1 through the generator line in 2 at the desired flow rate; a generator valve 4 for proportioning the saline flow between a radiopharmaceutical source like strontium-rubidium (⁸²Sr/⁸²Rb) generator 6 and a by pass line 7 which circumvents the generator 6; a positron detector 10 located downstream of the merge point 8 at which the generator and bypass flows merge; and a patient valve 12 for controlling supply of active saline to a patient outlet 17 and a waste reservoir. A controller 11 is connected to the pump 3, positron detector 10 and valves 5 and 5' and 12 and 12' to control the elution system 14 in accordance with a desired control algorithm. The controller may be connected to network, internet or other devices for communication or data exchange, monitoring *via* wired or wireless mediums.

The rubidium infusion system of the present invention may be used to assess various aspects of the system such as a concentration of ⁸²Rb, ⁸²Sr, or ⁸⁵Sr in a fluid that is eluted from the generator, the volume of the fluid that is eluted from the generator or the pressure of the fluid flowing through at least one portion of the system, volume of eluant, volume of waste bottle. Information about these aspects of the system may be gathered by various elements of the system and is sent to the controller. The controller may analyze this gathered data to assess the state of the system. In addition, the user interface computer may be configured to communicate with a remote computer, such as a server, network, other devices or a cloud computing service.

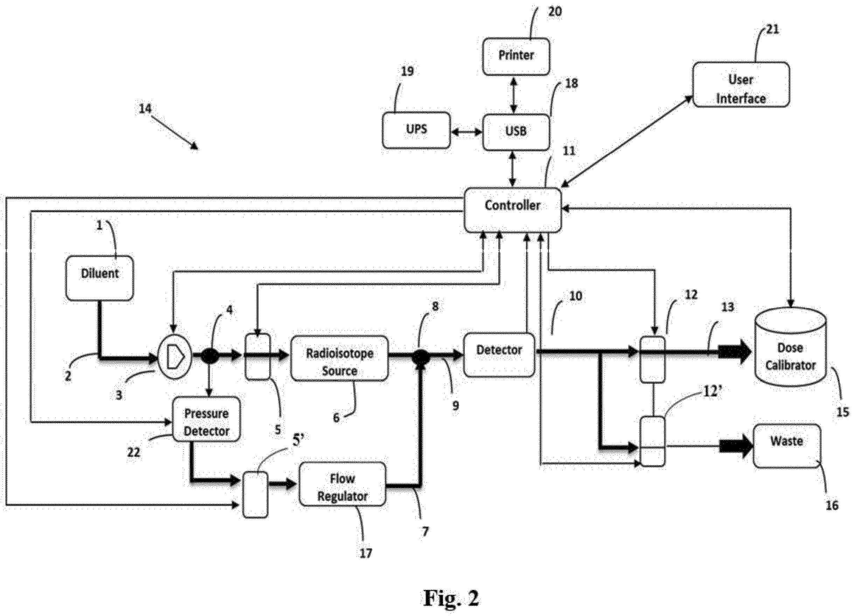

The elution system of **FIG. 2** may have a dose calibrator 15. The dose calibrator 15 may be used instead of a patient outlet, or in addition to a patient outlet, along with a valve that may be configured to direct fluid to the patient outlet or to the dose calibrator. The dose calibrator 15 may comprise a vial or container that collects the fluid as it otherwise exits the system. The dose calibrator 15 may be communicatively coupled to the controller, and configured to send information to the controller, such as an activity concentration of ⁸²Rb, ⁸²Sr, or ⁸⁵Sr in a fluid that is eluted from the generator or the volume of the fluid that is eluted from the generator. ⁸²Rb has a very short half-life of 76 seconds however ⁸²Sr, ⁸⁵Sr have half-lives of 25.36 and 64.853 days respectively. ⁸²Sr and ⁸⁵Sr in the eluate are result of breakthrough from the generator, which can have hazardous effects in case injected into patients because of longer half-lives at certain levels or above the threshold limit. Therefore, quality control tests for calibration and breakthrough are important aspects of rubidium infusion system.

According to some embodiments of the present disclosure, the computer of system includes monitor, which not only displays information of the system operation to inform the operator. This may comprise a touch screen (e.g. LED, LCD) to receive input from the operator. The computer is coupled to the controller of the infusion system. In alternate embodiments, a computer is coupled to the controller *via* a wire that allows the computer to be placed somewhat remotely to prevent radiation exposure to the operator. According to other embodiments, the computer is wirelessly coupled. According to some embodiments, the computer is pre-programmed to direct the operator *via* screen through procedures necessary to maintain, operate system, to perform quality control tests and for patient infusions. The computer may be programmed to interact with the controller of the system in order to keep a tally or count of elution per unit time for a given radiopharmaceutical generator. The elution counts along with measurements made on each sample or dose like activity level, volume, flow rate, pressure, errors may be maintained in a stored record on the computer. In alternate embodiments, the computer may be connected to other computers, infusion systems, pumps, dose calibrators, imaging devices, hospital networks, nursing stations, radiopharmaceutical generator manufacturers, infusion system manufacturers, authorized third parties responsible for management or maintenance of infusion system hardware and software components, personal computers, laptops, iPads, mobile tablet, mobile phones, wearable devices, watches and the like. In some embodiments of the present invention, the computer may be coupled with another device for operator data entry, examples of which include, without limitation, a peripheral keyboard device, keyboard, track ball, a storage medium, a reader, a scanner, a bar code reader or other reader of encoded information, mouse, joystick and like. The user interface computer may upload an indication of the result of the assessment to a computer *via* an authorized communications network. The remote computer may collect information from multiple computers and use this collected information to identify the state of a single infusion system or aggregate statistics for multiple ⁸²Sr/⁸²Rb infusion systems.

The controller of the device plays an important role in controlling and minimizing exposure to patients and operator. The controller is configured to perform mandatory quality control tests before infusion into the patient. As used herein, "quality control test" refers to the mandatory tests performed on daily basis for evaluating the safety and efficacy of elution system comprising a calibration to determine daughter isotope activity, a breakthrough testing to measure parent radioisotope activity in the sample. Quality control tests may further include, but are not limited to, checking and/or testing the status of generator columns, flow rate, pump speed, leakage, column and tubing pressure, activity of parent and daughter isotopes, sensors and valves functioning, checking the environment surrounding elution system, eluant type confirmation, volume of eluant, volume of waste bottle, testing outputs produced by columns and/or performing testing on samples of the radiopharmaceuticals produced by columns, scanning the system, network or attached devices for unauthorized connection and/or malware among other quality control measures. Quality control may be used to generate one or more quality reports which may include but are not limited to; analytical tests performed on the product; total yield of the products; failure reports for the product; failure reports for the one or more systems used to manufacture the product; and/or operator error reports. In an alternate embodiment, quality control results are displayed on the screen, provide audible and/or visual alerts/alarms to the operator. The alerts may be displayed on the user interface or can also be displayed on remote computer or system which is connected to the controller for remote operations of system. In another embodiment, the automated quality control tests can be performed in the night to save daytime or at predetermined time set by the operator.

In an embodiment, in addition to calibration and breakthrough testing, it is equally important to ensure that the system is free of any kind of cyber threat. In one embodiment, the quality control test comprises calibration, breakthrough testing, scanning for an unauthorized connection and/or malware in the system, network or connected devices. In an embodiment, in addition to calibration and breakthrough testing which determines the safety and efficacy of infusion system, a malware and/or an unauthorized connection scanning need to be performed during quality control before starting the infusions. In an alternate embodiment, a malware and/or an unauthorized connection scan can be performed continuously in the background and provide audible and/or visual alerts to the operator of any cyber threat. In an alternate embodiment, a malware and/or an unauthorized connection scan can be performed overnight or when the system is not eluting and the results of said malware and/or unauthorized scan is provided before the next system setup or elution. In such a scenario, the operator may have the option to repeat a malware scan and/or connection scan or accept the results obtained from overnight malware and/or an unauthorized connection scan. In response to detection of a malware or an unauthorized connection, the controller is configured to stop the patient elution or infusion until the threat is neutralized or allow the patient infusion in case system is found to be free of a malware or unauthorized connection. Rubidium generator requires about 10-15 minutes to be recharged after every elution, wherein the operator needs to wait for recharge of the generator for further elutions, therefore, in addition to confirmation of cybersecurity during quality control, an unauthorized connection and/or a malware scan can be performed during recharge time of the generator or when system is not eluting or administering into the patient. In an embodiment, the system comprises emergency stop button which may stop the system in case the cyber threat could be of potential concern. Pressing the emergency button will force the system into safe mode to prevent alterations in the set infusion parameters like dosing of radiopharmaceutical, calibration and breakthrough limits and data theft. In order to understand the severity of the threat, the controller is configured to provide a score to every cyber threat. Based on the severity, the controller may provide an alert and take necessary action for appropriate safety of the system, network devices, data and patients.

Calibration elution run enables the dose calibrator to be used to measure the generator performance in terms of ⁸²Rb activity concentration against eluted volume. This data can be used to predict eluted ⁸²Rb activity concentration for any given eluant flow rate. Repeating the calibration run at regular intervals allows the generator performance data to be updated to track changes in the generator performance as the generator ages and thereby enable accurate flow ratio prediction between successive calibration runs. If desired, calibration elutions can be scheduled to run automatically for example as part of a daily protocol which ensures system accuracy and at the same time limiting the potential for human error. The infusion system is calibrated by following procedure; the dose calibrator is automatically set for ⁸²Rb within the infusion system, the quality control test begins by automatically initiating a generator flush using about 40-95 mL, preferably about 75 mL of 0.9% Sodium Chloride Injection USP. This eluate is diverted towards the waste container and is ultimately discarded. After the generator flush, the system waits about 15 minutes to accomplish a complete generator recharge of ⁸²Rb half-lives. The system then elutes a calibration sample, using the dose calibrator, the system automatically quantifies the activity of ⁸²Rb in the calibration sample.

Breakthrough testing is another important part of quality control tests which is performed by using the calibration sample obtained from the ⁸²Rb eluate testing, the system allows the sample to stand for about 30 minutes to allow for the complete decay of ⁸²Rb. The system measures the activity of the sample to automatically determine the total ⁸²Sr and ⁸⁵Sr activity. The system automatically determines the ratio (R) on the day (post-calibration) of the measurement using the ratio of ⁸⁵Sr /⁸²Sr on the day of calibration provided on the generator label and the ⁸⁵Sr /⁸²Sr ratio factor from the ⁸⁵Sr /⁸²Sr ratio based on generator age. The system automatically determines if ⁸²Sr or ⁸⁵Sr in the eluate exceeds an alert or expiration limit. The expiration limit or threshold value is saved in the system memory for automatic alert and subsequent actions including not allowing patient infusion if measured value exceeds the threshold limit. A measurement of the concentration of a radioactivity relative to the applicable USP (United States Pharmacopeia) may be taken and actions can be taken based on this measurement. Where the measured value reaches a maximum threshold (for example, at least 50%) of the applicable USP standard, the system may be placed into a fail or error state and no further patient elutions performed until the generator has been replaced and/or an assessment shows that the concentration of a radioactivity relative to the USP is at an acceptable level. Where the measured value is less than a warning level of the applicable USP standard (for example, 20% thereof), elutions may occur normally and administered to patients for treatment or diagnosis. Where the measured value is between the warning and limit thresholds, a delimited number of patients (such as one to four patients) may be administered before additional assessment or calibration is required. The activity level of ⁸²Sr should not be more than 0.02 µCi per mCi of ⁸²Rb and ⁸⁵Sr is not more than 0.2 µCi per mCi of ⁸²Rb. In another embodiment, quality control testing may include checking the amount of undesired impurities in the radioactive eluate like Rubidium-83 (⁸³Rb) which should not be more than 0.02 µCi per mCi of ⁸²Rb.

The system is configured to ensure that system, network, connected devices are free of a malware and/or unauthorized connection. In case any unauthorized connection and/or malware is detected, the system will provide the alert and/or alarm to operator and halt further operations of the system. Halting the operations of the system may include automatic entry of system into safe mode which can prevent further operations, data theft, data changes, alternation in operations or specifications of system for patient safety until quality control tests comprising calibration, breakthrough, malware or connections are found to be acceptable and system is found to be safe for patient administration. The malware and connection scan can be performed prior to calibration and breakthrough testing or after calibration or breakthrough testing.

In an additional embodiment of the invention, the quality control tests are mandatory and cannot be evaded in any case. In case, the operator tries to evade any of the quality control test the controller is configured to halt the system for any further operations to enhance safety.

In an embodiment, the radiopharmaceutical infusion system comprises a eluant/diluent in a reservoir, a radioisotope source preferably generator containing a parent radioisotope that decays into daughter isotope, a first tubing line interconnecting the eluant reservoir and the generator, a pump for pumping the eluant from the eluant reservoir through said first tubing line, a first valve located on said tubing line and downstream the pump for directing the eluant to the generator or to a by-pass line, an eluate exiting the generator through a second tubing line and containing the daughter radioisotope, the second tubing line having a connection to receive the eluant from the by-pass line, a radioactivity detector on the second tubing line downstream said connection, a second valve on the second tubing line downstream the detector for directing the eluate to a patient line or to a waste line that is connected to a waste reservoir, the patient line is adapted for infusion into a patient, a controller for controlling the pump, the first valve and the second valve, and for receiving the information from the detector. In an alternate embodiment, the controller is connected to an imaging software of a radioisotope imaging device and quantification software for imaging the patient receiving the radioisotope infusion or a region of said patient and providing the qualitative and quantitative results automatically. The infusion system may be connected to one or more of hospital networks, pharmacies, manufacturers, operators, internet, servers, physicians, dose calibrators or others as the need arises.

In an embodiment, the terms "patient elution" and "patient infusion" are used interchangeably herein and refers to an eluate exiting the generator and containing the daughter radioisotope that is administered to a patient through intravenous or intra-arterial administration. In an embodiment, the terms "elution system" and "infusion system" are used interchangeably.

As used herein, the term cybersecurity refers to the practice of protecting systems, devices, networks and programs from digital attacks. These cyberattacks are usually aimed at accessing, changing, or destroying sensitive information; extorting money from users; or interrupting normal business processes. Vulnerabilities are weaknesses in computer software code, hardware designs, information systems, security procedures, internal controls that could be exploited by a threat. Some of the known vulnerabilities of cyber security include but not limited to authentication bypass, buffer overflow, code injection, communication protocol vulnerability, credentials insufficiently protected, cross-site scripting, cryptographic issues, data authenticity insufficiently verified, flash memory content insufficiently protected, hard-coded credentials, improper access control, improper authentication, improper authorization, improper certificate validation, improper control of generation code, improper exception handling, improper input validation, improper restriction of communication channel to intended endpoints, improper restriction of operations within the bounds of a memory buffer, leftover debug code, man in the middle, meltdown, spectre and spoiler, out-of-bounds read, path traversal, personal computer operating system vulnerabilities, protection mechanism failure, relative path traversal, resource consumption uncontrolled, resource management errors, session expiration insufficient, untrusted input accepted, vulnerable third-party software, xml external entity.

In one aspect, the system should be able to mitigate the threat *via* various controls of the system. This becomes especially important in case of radiopharmaceuticals, wherein the cyber-attacks on system should be controlled in appropriate manner to avoid any potential hazard. In case such threats are not mitigated or controlled the patient may be administered with the wrong dose of radiopharmaceutical and may lead to overexposure of radiation or lower dose of radiation which may provide wrong diagnosis or treatment as the case may be. In case of rubidium infusion system an excessive dose of ⁸²Sr or ⁸⁵Sr may cause adverse effects because both isotopes have longer half-life. Such exposure of long-acting radio isotopes may cause radiation hazard not only to patients, operators, hospital staff but also to people outside the medical facility if left undetected.

As used herein, the term 'malware' is malicious software intentionally designed to cause damage to a computer, server, client, or computer network, device. A wide variety of malware are known, including computer virus, worms, trojan horse, ransomware, spyware, adware, scareware, keylogger, mirai, reaper, emotet, bot, botnet and rootkit. Programs are also considered malware if they secretly act against the interests of the computer user. Malware in radiopharmaceutical device can alter the intended operations of the system like activity to be administered, dosing, flow rate, breakthrough or completely stop working of the system. Malware not only affects the system but also can affect other systems in the network and their operations.

There are various types of malware that may enter the system. These are also called as virus and designed to spread from host to host and has the ability to replicate itself. Similarly, in the same way that flu viruses cannot reproduce without a host cell, computer viruses cannot reproduce and spread without programming such as a file or document. In other words, a computer virus is a type of malicious code or program written to alter the way a computer operates and is designed to spread from one computer to another. A virus operates by inserting or attaching itself to a program or document that supports macros in order to execute its code. In the process, a virus has the potential to cause unexpected or damaging effects, such as harming the system software functioning by corrupting or destroying data. There are different types of virus or malware that may act differently to affect the system operations which include but are not limited to, boot sector virus can take control when you start or boot your system and may spread by plugging an infected USB drive into your computer. Web scripting virus exploits the code of web browsers and web pages, when you access such a web page, the virus can infect your computer. Browser hijacker hijacks certain web browser functions, and you may be automatically directed to an unintended website, Resident virus hat inserts itself in a computer system's memory and can execute anytime when an operating system load. Direct action virus comes into action when you execute a file containing a virus otherwise it remains dormant. Polymorphic virus changes its code each time an infected file is executed and does this to evade antivirus programs. File infector virus inserts malicious code into executable files used to perform certain functions or operations on a system. Multipartite virus infects and spreads in multiple ways and can infect both program files and system sectors. Macro virus are written in the same macro language used for software applications such viruses spread when you open an infected file.

Malware can be prevented by using anti-malware software, antispyware software, firewalls and Intrusion detection system (IDS), security scans, regular updates, scanning of every file coming *via* any authorized or unauthorized source. Firewall is one of the techniques, which can be useful in preventing malware. It can regulate traffic being sent out or received on devices, servers, network and/or web applications. They can block malicious traffic that tries to access the system, preventing hackers from successfully implanting malware into the system. In alternate embodiment, even if the system is infected with malware, firewalls may block the outgoing traffic that malware tries to use, stopping trojans and information stealers from transmitting stolen data back to hackers. Sandboxing is another method to prevent malware. It is used to protect computers and networks from more advanced threats that typically evade antivirus software. In this method, dedicated workstations are used to scan files, attachments, and storage devices for malware. Malware can be freely made to cause damage to the sandbox but since sandboxes are isolated devices, malware can be identified while being prevented from coming into contact with the rest of the network or radiopharmaceutical infusion system. Another method for malware protection includes content disarm and reconstruction. It sanitizes the files by removing just the malicious code and keeping the document usable. This method is capable of recovering potentially important information from the infected files. In an embodiment according to the present invention, one or more malware detection or defense techniques could be employed for scanning and neutralizing the malware threats to the systems.

Intrusion detection systems are one of the techniques, which are suitable for network security and detect any threats at an early stage. The detection system monitors and evaluates network activity to detect any unusual traffic. Intrusion detection systems can be based on artificial intelligence. An intrusion detection system (IDS) is an effective security technology, which can detect, prevent and react to the attacks. It monitors sources of activities, network traffic data in device computer or network systems and employs various techniques in order to provide security. Intrusion detection involves data acquisition, data preprocessing, model selection for data analysis and result analysis. In a preferred embodiment, the intrusion detection system is based on artificial intelligence. It provides the advantage to overcome the problem of false positives and advantageous for medical devices to minimize false positives and false alarms and disruptions in the systems. Another advantage of AI based systems is that AI's can learn new rules automatically, whereas in earlier systems the security manager adds new rules for each new type of threat or attack on the network or the system. AI based machine learning and deep learning are other important aspects for cybersecurity of radioisotope infusion systems and their connected networks. It is based on a large number of sample data, machines use pattern recognition to develop models that can be applied to new and unknown situations. AI based mechanisms like IDS, machine learning and deep learning can work in real time and provide round the clock protection to radiopharmaceutical infusion systems and networks associated with them. The cybersecurity provisions according to the present invention includes firewall, anti-virus, AI based software, machine learning and deep learning based software or hardware. The cybersecurity software can be embedded in the infusion system or can be located remotely. AI based software or algorithms are self-evolving and are automatically updated based on their experience of threat handling during operation. IDS can be Network Intrusion Detection Systems (NIDS) placed at a strategic point within the network to monitor traffic to and from all devices on the network. It performs an analysis of passing traffic and matches the traffic that is passed on the subnets to the library of known attacks. Once an attack is identified, the alert can be sent to the administrator/controller. NIDS can also be combined with other technologies to increase detection and prediction rates. Artificial Neural Network based IDS are capable of analyzing large volume of data in a smart way due to the self-organizing structure that allows it to more efficiently recognize intrusion patterns. Neural networks assist IDS in predicting attacks by learning from mistakes. Host intrusion detection systems (HIDS) run on individual hosts or devices on the network. A HIDS monitors the inbound and outbound packets from the device only and will alert the user or administrator if suspicious activity is detected. In another embodiment, detection method can be signature based or anomaly based.

In another embodiment, the radiopharmaceutical infusion systems are capable of securing the data backup automatically at predetermined interval of time. Backup is then transferred and stored to a cloud backup service. In other embodiment, data transfer can be performed in encrypted form. The predetermined interval of time according to this embodiment comprises an interval of 30 minutes, two hours, four hours, six hours, eight hours, ten hours, twelve hours, fourteen hours, sixteen hours, eighteen hours, twenty hours, twenty-two hours and twenty-four hours. In an alternate embodiment, data backup time can be set by the operator as per suitability as well. In another embodiment, in case any cyber threat is detected, the controller is configured to perform automatic data backup to a remote secure device, network or data cloud to safeguard the data.

In another embodiment, the malware protection can be multilayered security instead of a single layer for protection. More are the layers of defense, more difficult is for the hacker to access the device. Hence, the combination of various malware detection, protection and mitigation strategies are also included in the scope of the invention.

In another embodiment, if a malware and/or an unauthorized connection is detected, the controller is configured to repeat quality control to ensure the safety of the device. In such scenario, the system needs to repeat one or more tests like calibration, breakthrough testing, unauthorized connection check and malware check before operating the system.

As used herein, the term 'unauthorized connection' is a connection which is unrecognized and could not be approved by user, operator or system. Unauthorized connection may extract patient information, confidential information from the system or network. Unauthorized connection can be used to introduce malware or malicious software into the system. In an embodiment, the system comprises a database of IP addresses which are authorized to be used with the radiopharmaceutical elution system. The system is configured to identify any new IP address connection attempt to the system and alert the user. The system may also be configured to automatically discard unknown IP address connection requests. Unauthorized connection can be detected by network monitoring system which can detect the connection at the start of the system, during daily quality control, continuously during operations or at predetermined interval of time as determined by the user or during the recharge period of the generator. The predetermined time interval for automatic scanning may range from about 1 minute to 24 hours, like after 5 minutes, 10 minutes, 20 minutes, 30 minutes, 40 minutes, 50 minutes, 1 hour, 2 hours, 3 hours, 4 hours, 5 hours, 6 hours, 7 hours, 8 hours, 9 hours, 10 hours, 11 hours, 12 hours, 13 hours, 14 hours, 15 hours, 16 hours, 17 hours, 18 hours, 19 hours, 20 hours, 21 hours, 22 hours, 23 hours, 24 hours. The controller is programmed to scan according to predetermined set time interval for unauthorized connection or malware presence. In alternate embodiments, the scanning time intervals can be set by the operator as per convenience as well.

In an embodiment, the ideal network monitoring system should be automatic, capable of providing an immediate alert to the operator for risk assessment and remediation process. In one embodiment, the unauthorized connection can be wired or wireless. Wired connection includes but not limited to high definition multimedia interface (HDMI), radiofrequency (RF), video graphics array (VGA), digital visual interface (DVI), USB (Universal Serial Bus), Compact Disc (CD), Digital Video Disc (DVD) or combinations thereof. The Wireless connection includes but not limited to WIFI, Bluetooth, Infrared (IR), mobile phone, laptop, personal computer, computer tablet, iPad or combinations thereof.

The input/output source of the device may support input from and output to and from other devices that might couple to the computing device. This may, for example, include serial ports, Universal Serial Bus (USB) ports, optical ports, ethernet ports, telephone ports, audio jacks, component audio/video inputs, HDMI ports and so forth. This may also or instead include an infrared interface, RF interface, magnetic card reader, or other input/output system for coupling in a communicating relationship with other local devices. A USB port is used to attach to a WiFi accessory or where an ethernet connection is used to couple to a local network attached storage.

Embodiments of the invention may be implemented on an automated radiopharmaceutical infusion system that comprises a controller. The embodiments can be implemented to enhance the safety of radiopharmaceutical infusion/elution systems *via* automated mechanisms.

The embodiments of the invention described above are intended to be exemplary only.

## Claims

1. A 82Sr/82Rb radiopharmaceutical infusion system comprising a source of a radiopharmaceutical, a pump (3), an eluant in a reservoir (1), a waste bottle, a tubing line, a sensor, a valve, a controller (11), an emergency stop button wherein the source of the radiopharmaceutical comprises a generator column (6) containing a parent radioisotope that decays into a daughter radioisotope, wherein the pump is able to infuse an eluant through the generator column and provide a sample, wherein the controller is configured to perform quality control tests on the system comprising:
a) a connection scan on the system, on a network connected to the system or on a device connected to the system in order to confirm absence of an unauthorized connection;
b) a malware scan on the system, on a network connected to the system or on a device connected to the system in order to confirm absence of a malware;
c) system will send the alert to an operator on the detection of malware and/or unauthorised connection on the system;
d) a calibration of the sample to determine the daughter isotope activity;
e) a breakthrough test that measures the parent radioisotope activity in the sample;
wherein, the malware and/or the unauthorized connection scan on the system is performed and halt the system in case the operator tries to evade any of the quality control test.

2. The radiopharmaceutical infusion system according to claim 1, wherein the controller is configured to allow operation of the system after the breakthrough test and quality control test are performed automatically.

3. The radiopharmaceutical infusion system according to claim 1, further comprises additional quality control tests comprising verifications of the status of the generator column, a flow rate of the eluant, a pump speed, a leakage of the eluant, the type of the eluant, a volume in the waste bottle, the volume of eluant in the reservoir, the volume of eluant that has passed through the generator column the pressure in the tubing line, the activity of the parent isotope and the daughter isotope, sensor functioning and valve functioning.

4. The radiopharmaceutical infusion system according to claim 1, wherein the controller is further configured to perform a malware scan and/or an unauthorized connection scan continuously.

5. The radiopharmaceutical infusion system according to claim 1, wherein the malware and connection scan is performed overnight and results are displayed prior to breakthrough and calibration testing.

6. The radiopharmaceutical infusion system according to claim 1, wherein the controller is further configured to perform a malware scan and/or an unauthorized connection after an elution and the generator column is recharging and/or at a predetermined interval of time.

7. The radiopharmaceutical infusion system according to claim 1, further comprising an intrusion detection system, a firewall, or a sandboxing, wherein said intrusion detection system, firewall or sandboxing is embedded in the system or located remotely.

8. The radiopharmaceutical infusion system according to claim 7, wherein the intrusion detection system comprises a firewall, an anti-virus, an artificial intelligence based software, a learning machine or a deep learning based software or hardware.

9. The radiopharmaceutical infusion system according to claim 1, wherein the malware comprises a virus, a worm, a trojan horse, a ransomware, a spyware, an adware, a scareware, a keylogger, a mirai, reaper, an emotet, a bot, a botnet, a rootkit or a combination thereof.

10. The radiopharmaceutical infusion system of claim 1, wherein, upon detection of an attempt to connect the system, the controller is configured to:
a) provide an alert to a user about said attempt that requests confirmation of the authenticity of said attempt; and
b) stop the system from operating in case the user fails to authenticate the detected attempt to connect the system.

11. The radiopharmaceutical infusion system of claim 1, wherein the controller is configured to stop the system from operating in case a malware is detected by the malware scan until the malware is neutralized.

12. The radiopharmaceutical infusion system of claim 1, wherein the controller is configured to repeat the quality control tests in case a malware and/or an unauthorized connection is detected on the system.

13. A 82Sr/82Rb radiopharmaceutical infusion system comprising a source of a radiopharmaceutical, a pump (3), an eluant in a reservoir, a waste bottle, a tubing line, a sensor, a valve and a controller (11), wherein the source of the radiopharmaceutical comprises a generator column containing a parent radioisotope that decays into a daughter radioisotope, wherein the pump is able to infuse an eluant through the generator column (6) and provide a sample, wherein the controller is configured to perform a cyber threat detection comprising a step of:
a) a connection scan on the system, on a network connected to the system or on a device connected to the system in order to confirm absence of an unauthorized connection; and
b) a malware scan on the system, on a network connected to the system or on a device connected to the system in order to confirm absence of a malware;
c) an automatic data backup to a remote secure device, network or data cloud to safeguard the data if any cyber threat is detected;
d) providing a severity score to every detected cyber threat;
e) providing an alert to an operator and take necessary action for appropriate safety of the system, on the devices connected to system, data and patients based on the severity score; and
wherein the controller is configured to allow system operation once the absence of malware and/or an unauthorized connection is confirmed.

## Patentansprüche

1. Radiopharmazeutisches 82Sr/82Rb-Infusionssystem, umfassend eine Quelle eines Radiopharmazeutikums, eine Pumpe (3), ein Eluierungsmittel in einem Behälter (1), eine Abfallflasche, eine Schlauchleitung, einen Sensor, ein Ventil, eine Steuerung (11) und eine Not-Aus-Taste, wobei die Quelle des Radiopharmazeutikums eine Generatorsäule (6) umfasst, die ein Mutterradioisotop enthält, das in ein Tochterradioisotop zerfällt, wobei die Pumpe in der Lage ist, ein Eluierungsmittel durch die Generatorsäule zu infundieren und eine Probe bereitzustellen, wobei die Steuerung dazu konfiguriert ist, Qualitätssicherungstests an dem System durchzuführen, umfassend:
a) einen Verbindungsscan an dem System, an einem mit dem System verbundenen Netzwerk oder an einer mit dem System verbundenen Vorrichtung, um das Nichtvorhandensein einer nicht autorisierten Verbindung zu bestätigen;
b) einen Malware-Scan an dem System, an einem mit dem System verbundenen Netzwerk oder an einem mit dem System verbundenen Gerät, um das Nichtvorhandensein von Malware zu bestätigen;
c) das Senden der Warnung durch das System an eine Bedienperson, wenn Malware und/oder eine nicht autorisierte Verbindung im System erkannt wird;
d) eine Kalibrierung der Probe, um die Aktivität des Tochterisotops zu bestimmen;
e) einen Durchbruchtest, der die Aktivität des Mutterradioisotops in der Probe misst;
wobei der Scan auf Malware und/oder auf eine nicht autorisierte Verbindung an dem System ausgeführt wird und das System angehalten wird, falls die Bedienperson versucht, einen der Qualitätssicherungstests zu umgehen.

2. Radiopharmazeutisches Infusionssystem gemäß Anspruch 1, wobei die Steuerung dazu konfiguriert ist, den Betrieb des Systems zu erlauben, nachdem der Durchbruchtest und der Qualitätssicherungstest automatisch durchgeführt wurden.

3. Radiopharmazeutisches Infusionssystem gemäß Anspruch 1, ferner umfassend zusätzliche Qualitätssicherungstests, die Überprüfungen des Status der Generatorsäule, einer Durchflussrate des Eluierungsmittels, einer Pumpengeschwindigkeit, einer Leckage des Eluierungsmittels, des Typs des Eluierungsmittels, eines Volumens in der Abfallflasche, des Volumens des Eluierungsmittels im Behälter, des Volumens des Eluierungsmittels, das durch die Generatorsäule geflossen ist, des Drucks in der Schlauchleitung, der Aktivität des Mutterisotops und des Tochterisotops, der Funktionsweise des Sensors und der Funktionsweise des Ventils umfassen.

4. Radiopharmazeutisches Infusionssystem gemäß Anspruch 1, wobei die Steuerung ferner dazu konfiguriert ist, kontinuierlich einen Malware-Scan und/oder einen Scan auf eine nicht autorisierte Verbindung durchzuführen.

5. Radiopharmazeutisches Infusionssystem gemäß Anspruch 1, wobei der Malware- und Verbindungsscan über Nacht durchgeführt wird und Ergebnisse vor dem Durchbruch- und Kalibrierungstest angezeigt werden.

6. Radiopharmazeutisches Infusionssystem gemäß Anspruch 1, wobei die Steuerung ferner dazu konfiguriert ist, nach einer Eluierung und während des Aufladens der Generatorsäule und/oder in einem vorbestimmten Zeitintervall einen Malware-Scan und/oder eine nicht autorisierte Verbindung durchzuführen.

7. Radiopharmazeutisches Infusionssystem gemäß Anspruch 1, ferner umfassend ein Eindringungserkennungssystem, eine Firewall oder eine Sandbox, wobei das Eindringungserkennungssystem, die Firewall oder die Sandbox in das System eingebettet oder entfernt angeordnet ist.

8. Radiopharmazeutisches Infusionssystem gemäß Anspruch 7, wobei das Eindringungserkennungssystem eine Firewall, ein Antivirenprogramm, eine auf künstlicher Intelligenz basierende Software, eine lernende Maschine oder eine auf Deep Learning basierende Software oder Hardware umfasst.

9. Radiopharmazeutisches Infusionssystem gemäß Anspruch 1, wobei die Malware einen Virus, einen Wurm, ein Trojanisches Pferd, eine Ransomware, eine Spyware, eine Adware, eine Scareware, einen Keylogger, Mirai, Reaper, Emotet, einen Bot, ein Botnet, ein Rootkit oder eine Kombination davon umfasst.

10. Radiopharmazeutisches Infusionssystem gemäß Anspruch 1, wobei die Steuerung dazu konfiguriert ist, bei Erkennung eines Versuchs, eine Verbindung zum System herzustellen:
a) einem Benutzer eine Warnung über den Versuch bereitzustellen, die eine Bestätigung der Authentizität des Versuchs anfordert; und
b) den Betrieb des Systems zu stoppen, falls der Benutzer den erkannten Versuch, eine Verbindung zum System herzustellen, nicht authentifiziert.

11. Radiopharmazeutisches Infusionssystem gemäß Anspruch 1, wobei die Steuerung dazu konfiguriert ist, den Betrieb des Systems zu stoppen, falls durch den Malware-Scan eine Malware erkannt wird, bis die Malware neutralisiert ist.

12. Radiopharmazeutisches Infusionssystem gemäß Anspruch 1, wobei die Steuerung dazu konfiguriert ist, die Qualitätssicherungstests zu wiederholen, falls an dem System eine Malware und/oder eine nicht autorisierte Verbindung erkannt wird.

13. Radiopharmazeutisches 82Sr/82Rb-Infusionssystem, umfassend eine Quelle eines Radiopharmazeutikums, eine Pumpe (3), ein Eluierungsmittel in einem Behälter, eine Abfallflasche, eine Schlauchleitung, einen Sensor, ein Ventil und eine Steuerung (11), wobei die Quelle des Radiopharmazeutikums eine Generatorsäule umfasst, die ein Mutterradioisotop enthält, das in ein Tochterradioisotop zerfällt, wobei die Pumpe in der Lage ist, ein Eluierungsmittel durch die Generatorsäule (6) zu infundieren und eine Probe bereitzustellen, wobei die Steuerung dazu konfiguriert ist, eine Cyberbedrohungserkennung durchzuführen, umfassend einen Schritt:
a) eines Verbindungsscans an dem System, an einem mit dem System verbundenen Netzwerk oder an einer mit dem System verbundenen Vorrichtung, um das Nichtvorhandensein einer nicht autorisierten Verbindung zu bestätigen; und
b) eines Malware-Scans an dem System, an einem mit dem System verbundenen Netzwerk oder an einem mit dem System verbundenen Gerät, um das Nichtvorhandensein von Malware zu bestätigen;
c) einer automatischen Datensicherung auf einer entfernten sicheren Vorrichtung, einem Netzwerk oder einer Datencloud, um die Daten zu schützen, wenn eine Cyberbedrohung erkannt wird;
d) des Bereitstellens eines Schweregrads für jede erkannte Cyberbedrohung;
e) des Bereitstellens einer Warnung an eine Bedienperson und des Ergreifens der erforderlichen Maßnahmen für eine angemessene Sicherheit des Systems auf/in/an den mit dem System verbundenen Vorrichtungen, Daten und Patienten, basierend auf dem Schweregrad; und
wobei die Steuerung dazu konfiguriert ist, den Systembetrieb zu ermöglichen, sobald das Nichtvorhandensein von Malware und/oder einer nicht autorisierten Verbindung bestätigt ist.

## Revendications

1. Système de perfusion radiopharmaceutique 82Sr/82Rb comprenant une source d'un radiopharmaceutique, une pompe (3), un éluant dans un réservoir (1), un flacon à déchets, une ligne de tubulure, un capteur, une soupape, un contrôleur (11), un bouton d'arrêt d'urgence, dans lequel la source du radiopharmaceutique comprend une colonne de générateur (6) contenant un radio-isotope parent qui se désintègre en un radio-isotope fille, dans lequel la pompe est capable de perfuser un éluant à travers la colonne de générateur et de fournir un échantillon, dans lequel le contrôleur est configuré pour effectuer des tests de contrôle de qualité sur le système comprenant :
a) une analyse de connexion sur le système, sur un réseau connecté au système ou sur un dispositif connecté au système afin de confirmer l'absence d'une connexion non autorisée ;
b) une analyse de logiciel malveillant sur le système, sur un réseau connecté au système ou sur un dispositif connecté au système afin de confirmer l'absence d'un logiciel malveillant ;
c) le système enverra l'alerte à un opérateur lors de la détection de logiciel malveillant et/ou de connexion non autorisée sur le système ;
d) un étalonnage de l'échantillon pour déterminer l'activité de l'isotope fille ;
e) un test de percée qui mesure l'activité du radio-isotope parent dans l'échantillon ;
dans lequel, l'analyse de logiciel malveillant et/ou de connexion non autorisée sur le système est effectuée et arrête le système au cas où l'opérateur tente de se soustraire à l'un quelconque des tests de contrôle de qualité.

2. Système de perfusion radiopharmaceutique selon la revendication 1, dans lequel le contrôleur est configuré pour permettre l'opération du système après que le test de percée et le test de contrôle de qualité sont effectués automatiquement.

3. Système de perfusion radiopharmaceutique selon la revendication 1, comprend en outre des tests de contrôle de qualité supplémentaires comprenant des vérifications de l'état de la colonne de générateur, un débit de l'éluant, une vitesse de pompe, une fuite de l'éluant, le type de l'éluant, un volume dans le flacon à déchets, le volume d'éluant dans le réservoir, le volume d'éluant qui est passé à travers la colonne de générateur, la pression dans la ligne de tubulure, l'activité de l'isotope parent et de l'isotope fille, le fonctionnement du capteur et le fonctionnement de la soupape.

4. Système de perfusion radiopharmaceutique selon la revendication 1, dans lequel le contrôleur est en outre configuré pour effectuer en continu une analyse de logiciel malveillant et/ou une analyse de connexion non autorisée.

5. Système de perfusion radiopharmaceutique selon la revendication 1, dans lequel l'analyse de logiciel malveillant et de connexion est effectuée pendant la nuit et les résultats sont affichés avant les tests de percée et d'étalonnage.

6. Système de perfusion radiopharmaceutique selon la revendication 1, dans lequel le contrôleur est en outre configuré pour effectuer une analyse de logiciel malveillant et/ou une connexion non autorisée après une élution et que la colonne de générateur se recharge et/ou à un intervalle de temps prédéterminé.

7. Système de perfusion radiopharmaceutique selon la revendication 1, comprenant en outre un système de détection d'intrusion, un pare-feu, ou un bac à sable, dans lequel ledit système de détection d'intrusion, pare-feu ou bac à sable est intégré dans le système ou situé à distance.

8. Système de perfusion radiopharmaceutique selon la revendication 7, dans lequel le système de détection d'intrusion comprend un pare-feu, un antivirus, un logiciel basé sur l'intelligence artificielle, une machine d'apprentissage ou un logiciel ou matériel basé sur l'apprentissage profond.

9. Système de perfusion radiopharmaceutique selon la revendication 1, dans lequel le logiciel malveillant comprend un virus, un ver, un cheval de Troie, un rançongiciel, un logiciel espion, un logiciel publicitaire, un faux logiciel antivirus, un enregistreur de frappe, un Mirai, Reaper, un Emotet, un bot, un botnet, un logiciel passe-droit ou une combinaison de ceux-ci.

10. Système de perfusion radiopharmaceutique selon la revendication 1, dans lequel, lors de la détection d'une tentative de connexion au système, le contrôleur est configuré pour :
a) fournir une alerte à un utilisateur concernant ladite tentative qui demande une confirmation de l'authenticité de ladite tentative ; et
b) arrêter l'opération du système au cas où l'utilisateur ne parvienne pas à authentifier la tentative détectée de connexion au système.

11. Système de perfusion radiopharmaceutique selon la revendication 1, dans lequel le contrôleur est configuré pour arrêter l'opération du système au cas où un logiciel malveillant soit détecté par l'analyse de logiciel malveillant jusqu'à ce que le logiciel malveillant soit neutralisé.

12. Système de perfusion radiopharmaceutique selon la revendication 1, dans lequel le contrôleur est configuré pour répéter les tests de contrôle de qualité au cas où un logiciel malveillant et/ou une connexion non autorisée soient détectés sur le système.

13. Système de perfusion radiopharmaceutique 82Sr/82Rb comprenant une source d'un radiopharmaceutique, une pompe (3), un éluant dans un réservoir, un flacon à déchets, une ligne de tubulure, un capteur, une soupape et un contrôleur (11), dans lequel la source du radiopharmaceutique comprend une colonne de générateur contenant un radio-isotope parent qui se désintègre en un radio-isotope fille, dans lequel la pompe est capable de perfuser un éluant à travers la colonne de générateur (6) et de fournir un échantillon, dans lequel le contrôleur est configuré pour effectuer une détection de cybermenace comprenant une étape consistant en :
a) une analyse de connexion sur le système, sur un réseau connecté au système ou sur un dispositif connecté au système afin de confirmer l'absence d'une connexion non autorisée ; et
b) une analyse de logiciel malveillant sur le système, sur un réseau connecté au système ou sur un dispositif connecté au système afin de confirmer l'absence d'un logiciel malveillant ;
c) une sauvegarde automatique de données sur un dispositif sécurisé distant, un réseau ou un nuage de données pour protéger les données si une quelconque cybermenace est détectée ;
d) la fourniture d'un score de gravité à chaque cybermenace détectée ;
e) la fourniture d'une alerte à un opérateur et la prise de mesure nécessaire pour une sécurité appropriée du système, sur les dispositifs connectés au système, des données et des patients en fonction du score de gravité ; et
dans lequel le contrôleur est configuré pour permettre l'opération du système une fois que l'absence de logiciel malveillant et/ou d'une connexion non autorisée est confirmée.
